# EUROPEAN PATENT APPLICATION

(11) **EP 4 316 462 A1**
(43) Date of publication of application: **07.02.2024**
(21) Application number: 22189067.6
(22) Date of filing: 05.08.2022
(51) Int. Cl.: A61K 9/00, A61K 38/18, A61K 47/02, A61K 47/10, A61P 25/00, A61P 27/16

(54) **INTRANASAL ADMINISTRATION OF BDNF FOR THE TREATMENT OF SENSORINEURAL HEARING LOSS**

(71) Applicant: Dompé farmaceutici S.p.a., 20122 Milano (IT)
(72) Inventor: FETONI, Anna Rita, 00168 Roma (IT); ARAMINI, Andrea, 67100 L'Aquila (IT); BRANDOLINI, Laura, 67100 L'Aquila (IT); COCCHIARO, Pasquale, 67100 L'Aquila (IT); ROMEO, Tiziana, 60100 L'Aquila (IT); DETTA, Nicola, 80131 Napoli (IT); APPARENTE, Lucia, 67100 L'Aquila (IT); MATTIOLI, Simone, 67100 L'Aquila (IT); CATTANI, Franca, 67100 L'Aquila (IT); MANTELLI, Flavio, 67100 L'Aquila (IT); ALLEGRETTI, Marcello, 67100 L'Aquila (IT)
(74) Representative: Mauri, Elisabetta Maria Ester

(57) **Abstract**

The present invention relates to brain derived nerve factor (BDNF) for use in the prevention or treatment of sensorineural hearing loss in a subject, wherein said BDNF is administered intranasally to said subject.

## Description

### FIELD OF THE INVENTION

The present invention relates to the prevention and treatment of sensorineural hearing loss.

### STATE OF THE ART

Hearing loss is the fourth leading cause of disability worldwide, affecting 430 million people, over 5% of the of the world's population. It is estimated that by 2050 over 700 million people will have disabling hearing loss (World Health Organization, 2021 https://www.who.int/news-room/fact-sheets/detail/deafness-and-hearing-loss.).

The term hearing loss refers to a vast array of hearing disorders that can be classified into two main classes: (i) conductive hearing loss, wherein the hearing loss results from obstruction or disease of the outer or middle ear that prevents transmission of sound energy to the inner ear, and (ii) sensorineural hearing loss (SNHL), which constitute 90% of all cases of hearing loss, wherein the hearing loss results from damage to the inner ear and the auditory nerve. In particular, sensorineural hearing loss is mainly characterized by the degeneration of two types of cells: cochlear hair cells, which are the primary mechanoreceptors converting sound energy into neural signals, and/or auditory nerve neurons, the spiral ganglion cells (SGCs), that transmit signals from cochlear hair cells to the auditory nuclei of the brain stem through the auditory nerve.

Sensorineural hearing loss is usually initiated by degeneration of hair cells.

The above two types of cells are strictly connected, and a degeneration of sensory hair cells results in a decrease in synaptic signals stimulating spiral ganglion neurons, leading to neural degeneration (cochlear synaptopathy) (Cunningham al., N. Engl. J. Med. 2017, 377(25): 2465-2473).

The degeneration of hair cells in sensorineural hearing loss may have genetic or non-genetic etiology. Non-genetic factors include noise exposure, viral or bacterial infections, ototoxic chemicals such as the chemotherapeutic drug cisplatin or aminoglycoside antibiotics, autoimmune diseases and aging (Liu et al, Front. Neurosci. 2022, 16: art. 867453).

Despite the significant impact of hearing loss on all segments of the population, the only approved treatment option at the moment is cochlear cochlear implant (CI) auditory prosthesis, which functions by directly stimulating spiral ganglion cells (SGCs) soma and probably their central axons, delivering partial restoration of sensory organ function in patients. There are currently no pharmacological therapies that have been approved by FDA for the prevention and treatment of this family of disorders.

In clinical practice, non-genetic SNHL is usually treated by administration of corticosteroids, in particular dexamethasone and prednisolone. However, this treatment has not proven to lead to satisfactory outcomes.

It is therefore strongly felt the need to develop new, more effective, pharmacological therapies aimed et the inner ear and able to prevent damage or replenish hairs cells and neuron population in the cochlea and resotoring hearing function.

Brain-derived neurotrophic factor (BDNF) is a member of the neurotrophin family. This signaling molecule has been well-documented for its ability to regulate neuronal plasticity, cell growth, proliferation, cell survival, and long-term memory.

BDNF has been identified in preclinical and clinical studies as a promising therapeutic approach for the prevention and treatment of SNHL.

Preclinical studies have demonstrated that BDNF is effective in rescuing spiral ganglion neurons after hair cell damage. Also, in animal models of cochlear synaptopathy, local administration of BDNF to the cochlea has been shown to restore ribbon synapses and their function. (Liu et al, Front. Neurosci. 2022, 16: art. 867453, Foster at al, J. Acoust. Soc. Am. 2022, 151(6): 3937-3946, Foster at al, Pharmacol Res Perspect. 2022, 10(3): e00970).

Furthermore, clinical trials are ongoing to evaluate the safety, tolerability, and efficacy of OTO-413 administered as an intratympanic injection for the treatment of speech-in-noise hearing impairment (https://clinicaltrials.gov/ct2/show/NCT04129775?term=otonomy&draw=3).

However, the major challenge for the effective use of BDNF in the treatment of hearing disorders is the delivery of the protein to the inner ear compartment.

Tha delivery of drugs, particularly large molecules as proteins to the inner ear compartment is hindered by the presence of several protective barriers.

The blood labyrinth barrier (BLB), which separates the inner ear fluids from blood circulation, limits the possibility of systemic delivery. At the same time, delivery by local administration into the ear is hindered by the barriers between different compartments of the ear, the tympanic membrane (TM), between the outer and middle ear and the round window membrane (RWM), between the middle and inner ear.

To achieve greater drug concentrations in the middle ear, in the ongoing clinical trial using BDNF, the intratympanic injection of the protein has been used to deliver BDNF directly into the middle ear for subsequent diffusion into the inner ear across the RWM. However, this delivery route requires an invasive surgical procedure, which may distress patients and carries the risk of permanent tympanic membrane perforation.

Another approach used for the delivery of BDNF into the inner ear in preclinical studies has been the implantation of osmotic minipump into scala tympani. However, also in this case, the clinical application of this approach is limited by the fact that the patient has to undergo an invasive surgical procedure.

### SUMMARY OF THE INVENTION

The present inventors have now surprisingly found that BDNF can be delivered at effective concentrations in the inner ear by intranasal administration.

Accordingly, a first object of the invention is BDNF for use in the treatment of sensorineural hearing loss in a subject, wherein said BDNF is administered intranasally to said subject.

A further object of the invention is a pharmaceutical composition for intranasal administration comprising BDNF.

### BRIEF DESCRIPTION OF THE FIGURES

Figure 1 shows the concentration of BDNF in the perilymph at different timepoints after administration of rhBDNF (BDNF) or of phosphate buffer (PBS), measured as described in Example 1. ***: p< 0.0002 vs PBS; ****: p< 0.0001 vs PBS
Figure 2 shows the threshold shift of ABRs at low (6 kHz), mid (12, 16, and 20 kHz), and high (24 and 32 kHz) frequencies 7 days after the cisplatin treatment vs those measured at day 0, in mice treated with vehicle (grey line) or rhBDNF (black line), measured as described in Example 2.

### DETAILED DESCRIPTION OF THE INVENTION

A first object of the invention is brain derived nerve factor (BDNF) for use in the prevention or treatment of sensorineural hearing loss in a subject, wherein said BDNF is administered intranasally to said subject.

The terms "treatment" and "prevention" as used herein refer to the eradication/amelioration or prevention/delay in onset, respectively, of a disorder or of one or more of the symptoms associated thereof.

Preferably, said subject is a human subject.

According to one embodiment, said subject has been diagnosed with a sensorineural hearing loss and said BDNF is for use in the treatment of said sensorineural hearing loss by intranasal administration to said subject.

According to an alternative embodiment, said subject has been identified as being at risk of developing a sensorineural hearing loss and said BDNF is for use in the prevention of said sensorineural hearing loss by intranasal administration to said subject.

Preferably, said sensorineural hearing loss is a sensorineural hearing loss of non-genetic etiology.

Preferably, said sensorineural hearing loss of non genetic etiology is caused by noise exposure, a bacterial or viral infection, the treatment with an ototoxic drug, an autoimmune disease or aging.

Preferably, said ototoxic drug is selected from chemotherapeutic drugs and aminoglycoside antibiotics, more preferably it selected from cisplatin, caroboplatin and gentamicin.

Preferably said BDNF is human BDNF, more preferably it is recombinant human BDNF (rhBDNF).

Preferably, the BDNF for use according to the invention is administered from one to three times a day for a period of treatment of between 7 and 300 days, preferably between 60 and 240 days, more preferably between 100 and 200 days.

Preferably, the amount of BDNF per each administration is between 5 µg and 1 mg, more preferably between 10 µg and 400 µg, even more preferably between 15 µg and 200 µg. In case of treatment of SNHL, the effective amount of BDNF used in each administration, the duration of the treatment and the number of administrations for day are selected by the skilled person based on the characteristics of the subject to be treated, the severity of the SNHL and on the basis of the auditory tests carried out during the treatment.

In case of prevention of SNHL, the effective amount of BDNF used in each administration, the duration of the treatment and the number of administrations per day are selected by the skilled man based on the characteristics of the subject at risk of developing SNHL and evaluation of the entity and duration of the risk of developing SNHL as a result of one of said causes.

The evaluation of the above factors is within the knowledge and expertise of the skilled person.

A further object of the present invention relates to a pharmaceutical composition for intranasal administration comprising BDNF, as defined above.

Preferably, the pharmaceutical composition for intranasal administration of the invention is a liquid intranasal composition.

Preferably, the pharmaceutical composition according to the present invention comprises an effective amount of BDNF and at least one pharmaceutically acceptable excipient, preferably selected from solvents, thickening agents, mucoadhesive agents, buffers, antioxidants, preservatives, and penetration enhancers.

Preferably, the concentration of BDNF in the liquid intranasal composition according to the invention is between 5 µg/ml and 1 mg/ml, more preferably between 10 µg/ml and 400 µg/ml, even more preferably between 15 µg/ml and 200 µg/ml.

Preferably, said solvent is water.

Preferably, said antioxidant is methionine, more preferably at a concentration between 0.005 mg/ml and 0.02 mg/ml, more preferably of 0.01 mg/ml.

Preferably said surfactant is Kolliphor P188, more preferably at a concentration between 0.05 % w/v and 0.2% w/v, more preferably of 0.1% w/v.

Preferably, said buffer is phosphate buffer.

Preferably, said penetration enhancer is n-Dodecyl-β-D-maltoside, more preferably at a concentration between 0.1 % w/v and 1% w/v, more preferably of 0.5% w/v.

A particularly preferred liquid intranasal composition according to the invention comprises, preferably consists of, BDNF, sodium chloride, phosphate buffer and water. Another particularly preferred liquid intranasal composition according to the invention comprises, preferably consists of, BDNF, sodium chloride, phosphate buffer, Kolliphor P188, L-Methionine and water.

Another particularly preferred liquid intranasal composition according to the invention comprises, preferably consists of, BDNF, sodium chloride, phosphate buffer, Kolliphor P188, L-Methionine, n-Dodecyl-β-D-maltoside and water.

Preferably, liquid intranasal composition according to the invention comprises, preferably consists of, the following components:
- BDNF, preferably at a concentration between 5 µg/ml and 1 mg/ml, more preferably between 10 µg/ml and 400 µg/ml, even more preferably between 15 µg/ml and 200 µg/ml,
- NaH2PO4 * H2O, preferably at a concentration between 5 and 8 mg/ml, more preferably of 6.9 mg/mL,
- NaCl, preferably at a concentration between 5 and 6.5 mg/ml, more preferably of 5.84 mg/mL,
- Kolliphor P188, preferably at a concentration between 0.05 % w/v and 0.2% w/v, more preferably of 0.1% w/v,
- L-Methionine, preferably at a concentration between 0.05 mg/ml and 0.2 mg/ml, more preferably of 0.1 mg/ml,
- Optionally, n-Dodecyl-β-D-maltoside, preferably at a concentration between 0.1 % w/v and 1% w/v, more preferably of 0.5% w/v,
- Water.

The pharmaceutical composition according to the invention may be suitably formulated using appropriate methods known in the art or by the method disclosed in Remington's Pharmaceutical Science (recent edition), Mack Publishing Company, Easton Pa. Preferably, the pharmaceutical composition of the invention is for use in the prevention or treatment of sensorineural hearing loss in a subject, as above described, wherein said formulation is administered to the subject intranasally.

In a further aspect, the present invention relates to a method for the prevention or treatment of sensorineural hearing loss in a subject comprising intranasally administering to the subject BDNF, as described above, in a therapeutically effective amount. Preferably, in the method according to the invention, said BDNF is administered as described above.

Preferably, said BDNF used in the method of the invention is in form of a pharmaceutical composition, as above defined.

The invention will be further described in the following examples, which do not limit the scope of the invention defined in the claims.

### EXPERIMENTAL SECTION

### Example 1

The bioavailability of recombinant human BDNF (rhBDNF) in the inner ear (perilymph) after a single intranasal administration was evaluated in 10 week old C57BL6 mice (Envigo Labs, France).

The mice were divided in six groups:
Group 1 (n=4): control group, treated with PBS and sampling carried out at 2 hours after treatment.
Groups 2-6: rhBDNF-treated groups with sampling of the periplymph at different timepoints:
   Group 2 (n=4): treated with rhBDNF and sampling carried out at 2 hours.
   Group 3 (n=4): treated with rhBDNF and sampling carried out at 6 hours.
   Group 4 (n=4): treated with rhBDNF and sampling carried out at 12 hours.
   Group 5 (n=4): treated with rhBDNF and sampling carried out at 24 hours.
   Group 6 (n=4): treated with rhBDNF and sampling carried out at 48 hours.

The animals were treated as follows.

A micropipette was filled with 10 µl of a solution of rhBDNF (expressed in E.coli at Dompé farmaceutici SpA) at 2mg/ml in sodium phosphate buffer 50mM, 100mM NaCl, pH 7.2, (groups 2-6) or with PBS (group 1) and the tip of the filled pipette was placed near the mouse's left nostril, which was kept at a 45 degree angle, and a droplet of the content released which was immediately inhaled by the mouse. A second droplet was released for the mouse to inhale it through the same nostril about 2-3 sec later. After administration, the mice were held in this position for 15 sec.

All mice were sacrificed by cervical dislocation at the relevant timepoints and the tympanic bullas were isolated. Each tympanic bulla was opened, the cochlea was cleaned with PBS and 1 µl of cochlear perilymph was sampled from each cochlea using a capillary micropipette and immediately frozen in a low-binding Eppendorf tube and stored at -20 °C before ELISA analysis. The contralateral cochlea was used as duplicated.

The cochlear perilymph samples were diluted at 1/10 and 10 µl per well analyzed by ELISA (Thermo Fisher, Ref. EH42RB).

Dilutions of the rhBDNF standard reference were performed at 0, 0.066 ng/mL, 0.160 ng/mL, 0.410 ng/mL, 1.020 ng/mL, 2.560 ng/mL, 6.40 ng/mL and 16 ng/mL to perform the standard curve. The standard curve of rhBDNF obtained presented a linear regression of R square 0.9997 and slope of 0.1209 ± 0.0005206 allowing to perform the interpolation of the curve to determine the rhBDNF concentration in each mouse sample.

The standard working solution of different concentrations was added to the first two columns: each concentration of the solution was added into two wells side by side and the samples to be analyzed to the other wells (10 µl/well). The plate was covered with sealer provided in the kit and incubated overnight at 4°C. The washing and incubation procedure was performed following the manufaturer's instructions. At the end of the procedure 50 µl of Stop Solution was added to each well and the optical density of each well was immediately determined using a micro-plate reader set to 450 nm.

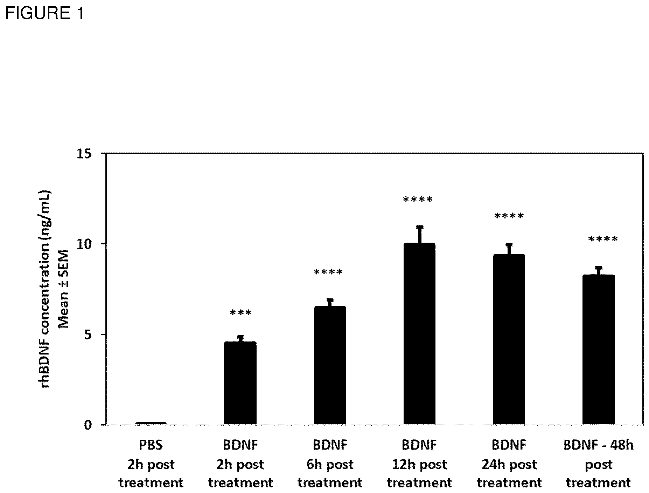

The results of the analysis are reported in Figure 1.

As can be seen from Figure 1, a progressive and significant increase of rhBDNF concentration was observed in perilymph from 2 hours to 48 hours post-treatment in the rhBDNF treated group compared to the PBS treated control group. The concentration of rhBNDF reached a peak at 12 hours post administration with a concentration of 9.91 ng/mL ± 1.02. Then, a decrease of rhBDNF concentration was observed at 24 hours and 48 hours post-treatment with a concentration (Mean±SEM) of 9.33 ng/mL ± 0.59 and 8.20 ng/mL ± 0.47 respectively. The data obtained demonstrate that intranasal administration of rhBDNF results in delivery of the protein in the cochlear perilymph with a peak concentration at 12 hours post treatment and an accumulation until 48 hours post treatment, suggestinf that this route of administration is suitable to target hearing disorders.

### Example 2

The efficacy of recombinant human BDNF (rhBDNF) administered via the intranasal route was tested in cisplatin induced hearing loss mouse model.

In details, male adult Wistar rats (Catholic University Laboratories, 200-250 g), 2 months of age, with normal Preyer's reflex, were used in this study. The experiments were performed on a total of 13 animals, randomized and assigned to two experimental groups: Group 1: animals treated with cisplatin and phosphate buffer pH 7.2 (vehicle); ("Cis + vehicle" group; n = 6),

Group 2: animals treated with cisplatin and recombinant hBDNF ("Cis + rhBDNF" group; n = 7).

All animals were treated with cisplatin (Cat. No. P4394, Sigma-Aldrich, St. Louis, MO, USA) at time 0. In details, cisplatin was diluted in sterile saline (1 mg/ml), and prepared freshly, protected by light. To facilitate drug dissolution, the solution was heated and stirred for a period of 20 minutes. Under deep anesthesia, a single cisplatin dose of 12 mg/kg was delivered intraperitoneally (i.p.) at a rate of 8 ml/h with an infusion pump (Axon Instruments, Foster City, CA, USA) over about 30 minutes. The animals were hyper-hydrated with saline solution (subcutaneous injection, 15 ml daily) to limit cisplatin side effects.

rhBDNF (expressed in *E.coli* at Dompé farmaceutici SpA) (2.4mg/mL) (Group 2) or vehicle (Group 1) was then administered by intra-nasal route daily for 7 days, starting 1 hour after the cisplatin treatment (day 0). Using a dominant hand (without anesthesia), the micropipette (Gilson) was filled with 40µl of rhBDNF or vehicle. The tip of the filled pipette was placed in the rat's nostril, kept at a 45-degree angle. Each administration involved the gradual release of two single drops of the drug or vehicle in the nostrils (40µl per nostril, 80µl animal). The drops were sucked up by the animal taking care to prevent its transfer into the oral opening. The immobilized animal was positioned in a supine-like position to facilitate the entry and absorption of the drug in the nostril. At the end of each administration, the rat was placed in its own cage to allow the complete drying of the nostril.

In order to assess hearing loss induced by cisplatin treatment and the effect of rhBDNF administration, hearing function was estimated by ABR recordings. In all animals, ABRs were measured at low (6 kHz), mid (12, 16, and 20 kHz), and high (24 and 32 kHz) frequencies at day 0 and 7 days from treatment onset. In all animals, ABRs were assessed bilaterally before treatment to assure normal hearing and reassessed at all-time points to evaluate the effect of treatments on hearing.

The ABRs recordings were performed as follows. All animals were mildly anesthetized (ketamine, 35 mg/kg and medetomidine-domitor, 0.25 mg/kg) and placed in the anechoic room. Three stainless steel recording electrodes were subcutaneously inserted posterior to the tested pinna (active), vertex (reference), and contralateral pinna (ground). A PC-controlled TDT System 3 (Tucker Davis Technologies, Alachua, FL, USA) data acquisition system with real-time digital signal processing was used for ABR recording and auditory stimulus generation. Tone bursts of pure tones from 6 to 32 kHz (1 ms rise/fall time, 10 ms total duration, 20/s repetition rate) were presented monaurally. Responses were filtered (0.3-3 kHz), digitized, and averaged (across 500 discrete samples at each frequency-level combination). Threshold value was defined as the lowest stimulus level that yielded a repeatable waveform-based onset.

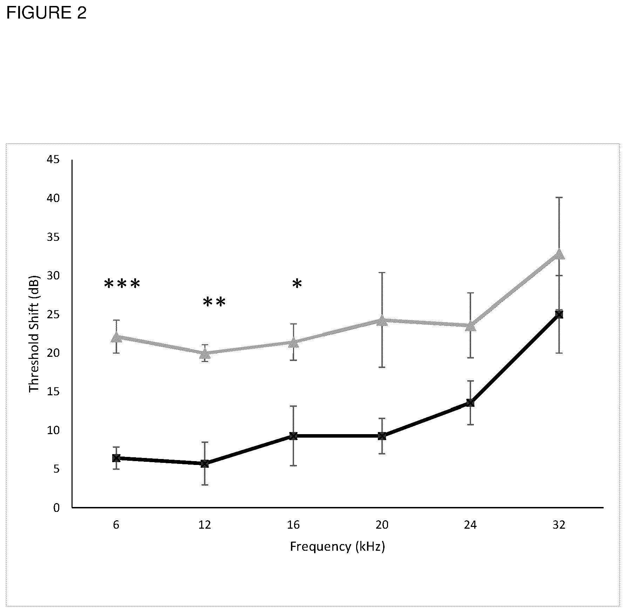

The results obtained are shown in Figure 2.

As can be seen, in the group treated with cisplatin and vehicle an increase of threshold shift of about 25 dB in all frequencies analysed was observed, while the treatment with rhBDNF reduced hearing loss by approximately 15 dB in the low and mid frequencies and 10 dB in the high frequencies.

These data demonstrate that the non-invasive intranasal administration of rhBDNF induced a recovery of auditory function in an animal model of cisplatin-ototoxicity.

## Claims

1. Brain derived nerve factor (BDNF) for use in the prevention or treatment of sensorineural hearing loss in a subject, wherein said BDNF is administered intranasally to said subject.

2. BDNF for use as claimed in claim 1, wherein said sensorineural hearing loss is of non-genetic etiology.

3. BDNF for use as claimed in claim 2, wherein said sensorineural hearing loss of non-genetic etiology is caused by by noise exposure, a bacterial or viral infection, the treatment with an ototoxic drug, an autoimmune disease or aging.

4. BDNF for use as claimed in claims 1 to 3, wherein said BDNF is human BDNF, more preferably it is recombinant human BDNF.

5. BDNF for use as claimed in claims 1 to 4, wherein said BDNF is administered from one to three times a day for a period of treatment of between 7 and 300 days, preferably between 60 and 240 days, more preferably between 100 and 200 days.

6. BDNF for use as claimed in claims 1 to 5, wherein the amount of BDNF per each administration is between 5 µg and 1 mg, more preferably between 10 µg and 400 µg, even more preferably between 15 µg and 200 µg.

7. A pharmaceutical composition for intranasal administration comprising BDNF and at least one pharmaceutically acceptable excipient.

8. A pharmaceutical composition as claimed in claim 7, wherein said BDNF is human BDNF, more preferably it is recombinant human BDNF.

9. A pharmaceutical composition as claimed in claim 7 or 8, wherein said BDNF is contained in the composition at a concentration between 5 µg/ml and 1 mg/ml, more preferably between 10 µg/ml and 400 µg/ml, even more preferably between 15 µg/ml and 200 µg/ml.

10. A pharmaceutical composition as claimed in claims 7 to 9, comprising, preferably consisting of BDNF, sodium chloride, phosphate buffer and water.

11. A pharmaceutical composition as claimed in claim 7 to 9, comprising, preferably consisting of:
- BDNF, preferably at a concentration between 0 between 5 µg/ml and 1 mg/ml, more preferably between 10 µg/ml and 400 µg/ml, even more preferably between 15 µg/ml and 200 µg/ml.,
- NaH2PO4 * H2O, preferably at a concentration between 5 and 8 mg/ml, more preferably of 6.9 mg/mL,
- NaCl, preferably at a concentration between 5 and 6.5 mg/ml, more preferably of 5.84 mg/mL,
- Kolliphor P188, preferably at a concentration between 0.05 % w/v and 0.2% w/v, more preferably of 0.1% w/v,
- L-Methionine, preferably at a concentration between 0.05 mg/ml and 0.2 mg/ml, more preferably of 0.1 mg/ml,
- Optionally, n-Dodecyl-β-D-maltoside, preferably at a concentration between 0.1 % w/v and 1% w/v, more preferably of 0.5% w/v,
- Water.

12. A pharmaceutical composition as claimed in claims 1 to 11, wherein said BDNF is human BDNF, more preferably it is recombinant human BDNF.

13. A pharmaceutical composition as claimed in claims 1 to 12, for use in the prevention or treatment of sensorineural hearing loss.

14. A pharmaceutical composition for use as claimed in claim 13, wherein said sensorineural hearing loss of non-genetic etiology is caused by noise exposure, a bacterial or viral infection, the treatment with an ototoxic drug, an autoimmune disease or aging.
